# FASCICULE DE BREVET EUROPEEN

(11) **EP 3 445 432 B1**
(45) Date de publication et mention de la délivrance du brevet: **20.11.2019**
(21) Numéro de dépôt: 17725295.4
(22) Date de dépôt: 20.04.2017
(51) Int. Cl.: A61M 16/08, A61M 16/04, A61M 16/12, A61B 5/097

(54) **CATHÉTER DE MESURES MIXTES POUR SONDE D'INTUBATION RESPIRATOIRE**
KATHETER ZUR ERMÖGLICHUNG KOMBINIERTER MESSUNGEN FÜR EINE BEATMUNGSINTUBATIONSSONDE
CATHETER PERMITTING COMBINED MEASUREMENTS FOR A RESPIRATORY INTUBATION PROBE

(30) Priorité: 22.04.2016 FR 1653601
(43) Date de publication de la demande: 27.02.2019
(73) Titulaire: Boussignac, Georges, 92160 Antony (FR)
(72) Inventeur: Boussignac, Georges, 92160 Antony (FR)
(74) Mandataire: Jacobacci Coralis Harle
(86) Numéro de dépôt international: PCT/FR2017/050937
(87) Numéro de publication internationale: WO 2017/182757

(56) Documents cités:
- EP-A1- 0 888 792
- EP-A2- 2 140 901
- WO-A1-01/23025
- WO-A1-2015/052488
- WO-A1-2015/189525
- WO-A2-02/13885
- WO-A2-2006/070366
- DE-B3-102006 023 273
- FR-A1- 2 770 137
- FR-A1- 2 813 197
- FR-A1- 2 827 778
- US-A- 5 515 844
- US-A- 5 954 050

## Description

### DOMAINE TECHNIQUE AUQUEL SE RAPPORTE L'INVENTION

La présente invention concerne de manière générale le domaine des appareils d'assistance respiratoire. Elle concerne plus particulièrement un cathéter de mesures mixtes destiné à une sonde d'intubation respiratoire pour une utilisation particulièrement utile en condition de réanimation cardiopulmonaire d'une personne en arrêt cardiorespiratoire.

### ARRIERE-PLAN TECHNOLOGIQUE

Des études récentes ont montré l'intérêt dans la réanimation d'urgence de personnes en arrêt cardiorespiratoire, d'une insufflation continue d'oxygène en parallèle d'un massage thoracique cardiaque continu le plus tôt possible. En effet, la méthode traditionnelle de réanimation dans laquelle le massage thoracique cardiaque est interrompu pour permettre des insufflations d'oxygène présente le grave défaut de ramener à zéro la circulation sanguine pendant les pauses du massage et de nécessiter un certain temps de réamorçage pour revenir à des conditions hémodynamiques quelque peu satisfaisantes après la reprise du massage. En outre, la ventilation doit être particulièrement forcée du fait de son intermittence, ce qui crée d'autres problèmes tant circulatoires que respiratoires.

L'insufflation continue d'oxygène nécessite préférentiellement un dispositif d'assistance respiratoire, notamment masque facial ou tube/sonde d'intubation respiratoire, qui soit du type ouvert pour éviter des surpressions dans les voies respiratoires. Les dispositifs d'assistance respiratoire à jets défléchis de Monsieur BOUSSIGNAC et leurs dérivés à injection continue d'oxygène sont particulièrement intéressants dans cette optique. Ceux dont les espaces morts sont les plus réduits sont encore plus intéressants comme par exemple les tubes/sondes d'intubation respiratoire dans lesquelles l'oxygène est injecté en jets défléchis vers l'extrémité distale de la sonde, c'est-à-dire du côté de la personne/ de ses poumons. Les tubes/sondes d'intubation respiratoire sont notamment des sondes d'intubation trachéale ou des sondes à masques pharyngés ou laryngés.

Parmi les dispositifs de Monsieur BOUSSIGNAC, on peut citer ceux décrits dans les documents FR2012/58034 (FR 2 994 852 A1), FR2011 /01035 (FR 2 973 708 A1), FR2009/01752 (FR 2 944 210 A1), FR2011 /00338 (FR 2 971 162 A1), FR2009/04364 (FR 2 942 966 A1), FR2007/07036 (FR 2 921 840 A1), EP99 401 804 (EP 0 978 291 A1) et EP02 788 034 (EP 1 441 791 A1).

En particulier, le dispositif d'intubation décrit dans FR2010/03343 (FR 2 963 740 A1) peut être utilisé utilement en réanimation du fait de ses différentes possibilités de connexion.

L'utilisation de ces dispositifs se fait dans l'urgence, dans des conditions de terrain, et les opérateurs, notamment les pompiers, doivent gérer de nombreux paramètres lors de la réanimation. En particulier, il faut s'assurer que la ventilation est efficace tout comme le massage thoracique. Il est donc mis en œuvre des appareils de mesure de ces paramètres notamment grâce à un manomètre pour mesurer la pression dans le dispositif d'assistance respiratoire et à une mesure de la composition/concentration des gaz respiratoires. Concernant la composition/concentration des gaz respiratoires, la mesure du CO₂ est un indicateur intéressant de la circulation sanguine (outre de la qualité des échanges gazeux dans les poumons). Ces appareils de mesure sont actuellement disposés à l'extérieur de la personne, côté proximal (côté opposé à la personne) du dispositif d'assistance respiratoire et il est donc nécessaire de les connecter au dispositif d'assistance respiratoire pour surveiller l'hémodynamique et la ventilation.

L'utilité de la surveillance de ces paramètres est telle qu'il s'est dégagé un consensus dans les milieux concernés pour recommander, voir imposer, au moins la mesure du CO₂ lors du massage thoracique continu sous insufflation continue d'oxygène.

La difficulté qui se présente alors est le fait que de l'oxygène est injecté dans le dispositif d'assistance respiratoire, ce qui perturbe les mesures de concentration gazeuse sauf à prévoir un prélèvement distal (du côté de la personne) du dispositif d'assistance respiratoire. C'est ainsi que certains de ces dispositifs d'assistance respiratoire prévoient une microtubulure dans l'épaisseur de leur paroi et qui débouche côté distal du dispositif. Du fait des dimensions des dispositifs d'assistance respiratoire, ces microtubulures ont un diamètre très réduit qui ne peut pas être augmenté sauf à réduire la lumière centrale utile du dispositif d'assistance respiratoire et donc gêner la ventilation que l'on souhaite justement restaurer. En pratique ces microtubulures ne permettent pas une mesure de la pression et, de plus, ne permettent pas des mesures correctes de la composition pour les appareils de mesure passifs (sans aspiration) du fait du faible débit gazeux possible en circulation passive dans la microtubulure. Or ce sont ces appareils de mesure simples, passifs, qui sont utilisés sur le terrain, dans l'urgence, du fait de leurs dimensions réduites et de leur faible consommation.

En pratique, les dispositifs d'assistance respiratoire ne comportent pas de moyens simples et efficaces permettant des mesures correctes à la fois de pression et de composition gazeuse. Ceux permettant certaines mesures nécessitent la création de structures complexes qui ne seront finalement peut être pas utilisées, par exemple du fait que les appareils de mesure, notamment passifs pour la composition gazeuse, ne le permette pas ou qu'ils sont utilisés dans d'autres conditions que la réanimation d'urgence. En effet, certains dispositifs d'assistance respiratoire sont configurés pour être quasi universels et être utilisés dans diverses conditions et pour divers buts, ce qui conduit à des compromis dans leurs structures et par exemple les dimensions ou le nombre de voies gazeuses.

On connait par le document EP 2 140 901 A2 un dispositif auxiliaire respiratoire comportant deux tubes principaux dont un comporte un tube supplémentaire de mesure de pression. Les documents WO 02/13885 A2, WO 01/23025 A1 et EP 0 888 792 A1 divulguent des moyens d'intubation avec mesure de la pression. Le document DE 10 2006 023 273 B3 divulgue un système d'intubation avec mesure de la composition des gaz. Les documents FR 2 827 778 A1 et FR 2 770 137 A1 divulguent des dispositifs d'assistance respiratoire à jets défléchis formant une soupape virtuelle.

### OBJET DE L'INVENTION

Afin de remédier à l'inconvénient précité de l'état de la technique, la présente invention propose une solution qui permet d'utiliser les dispositifs d'assistance respiratoire connus ou à venir dans leur fonction de base de liaison aux voies respiratoires de la personne tout en permettant d'effectuer des mesures en toute ou quasi toute indépendance de la structure du dispositif. On verra même que cette solution permet, dans une version évoluée, de compléter la fonction de base de liaison avec une fonction d'injection d'oxygène avec espace mort réduit.

Cette solution consiste en un cathéter pour mesures mixtes destiné à être introduit et passé dans la lumière, dite canal principal, d'un dispositif d'assistance respiratoire, ledit dispositif d'assistance respiratoire comportant une extrémité distale destinée à être placée sur ou introduite dans les voies respiratoires d'une personne et une extrémité proximale destinée à être disposée à l'extérieur de la personne et qui est opposée à l'extrémité distale, ledit cathéter ayant une extrémité proximale (du côté opposé à la personne) et une extrémité distale (du côté de la personne et de la trachée) et étant configuré pour que, une fois installé sur le dispositif d'assistance respiratoire, son extrémité distale se positionne au-delà de l'extrémité distale du dispositif d'assistance respiratoire ou, à tout le moins, côté distal d'un/de jets d'oxygène injecté dans la lumière du dispositif d'assistance respiratoire dans le cas où le dispositif d'assistance respiratoire comporterait des moyens d'injection d'oxygène dans sa lumière, l'extrémité distale du cathéter pouvant alors se trouver dans le dispositif d'assistance respiratoire.

Plus particulièrement, on propose selon l'invention un cathéter de mesures mixtes destiné à être introduit et passé dans un canal principal interne de circulation de gaz respiratoires d'un dispositif d'assistance respiratoire, ledit dispositif d'assistance respiratoire comportant une extrémité distale destinée à être placée sur ou introduite dans les voies respiratoires d'une personne et une extrémité proximale destinée à être disposée à l'extérieur de la personne et opposée à l'extrémité distale, ledit cathéter ayant une extrémité proximale (du côté opposé à la personne) et une extrémité distale (du côté de la personne et de la trachée) entre lesquelles s'étend intérieurement une lumière dudit cathéter, ledit cathéter comportant a son extrémité proximale, d'une part, un dispositif de connexion à l'extrémité proximale du dispositif d'assistance respiratoire et, d'autre part, une liaison gazeuse de la lumière du cathéter à un appareil de mesure, le cathéter étant configuré pour que, une fois introduit dans le canal principal et connecté au dispositif d'assistance respiratoire, son extrémité distale se positionne vers l'extrémité distale du dispositif d'assistance respiratoire ou, à tout le moins, du côté distal d'un/de jets d'oxygène injecté dans la lumière du dispositif d'assistance respiratoire dans le cas où le dispositif d'assistance respiratoire comporterait des moyens d'injection d'oxygène dans son canal principal.

Selon l'invention, ledit cathéter comporte a son extrémité proximale un raccord de liaison gazeuse de la lumière du cathéter à un appareil de mesure de composition gazeuse et à un appareil de mesure de pression gazeuse, et dans le raccord de liaison gazeuse, la liaison gazeuse pour l'appareil de mesure de composition gazeuse présente une section de passage plus réduite que la section de passage de la liaison gazeuse pour l'appareil de mesure de pression.

D'autres caractéristiques non limitatives et avantageuses du cathéter conforme à l'invention, prises individuellement ou selon toutes les combinaisons techniquement possibles, sont les suivantes :
- l'oxygène injecté est pur,
- l'oxygène injecté fait partie d'une composition de gaz respirable injecté,
- l'extrémité distale du cathéter se positionne et débouche vers l'extrémité distale du dispositif d'assistance respiratoire dans le canal principal de ce dernier,
- l'extrémité distale du cathéter se positionne et débouche au niveau ou au-delà de l'extrémité distale du dispositif d'assistance respiratoire,
- l'appareil de mesure de composition gazeuse permet la mesure au moins du CO₂,
- l'appareil de mesure de composition gazeuse comporte une entrée et une sortie et la réduction de la section de passage pour l'appareil de mesure de composition gazeuse est du côté de l'entrée de l'appareil de mesure de composition gazeuse,
- l'appareil de mesure de composition gazeuse comporte une entrée et une sortie et la réduction de la section de passage pour l'appareil de mesure de composition gazeuse est du côté de la sortie de l'appareil de mesure de composition gazeuse,
- la réduction de la section de passage pour l'appareil de mesure de composition gazeuse est obtenue par un étranglement d'une tubulure de liaison,
- la réduction de la section de passage pour l'appareil de mesure de composition gazeuse est obtenue par une bague de restriction disposée dans une tubulure de liaison,
- la tubulure de liaison est un embout d'un robinet trois voies, la bague de restriction étant disposée dans ledit embout dudit robinet,
- les deux appareils de mesure sont reliés en parallèle à la lumière du cathéter, le raccord de liaison gazeuse aux deux appareils de mesure comportant deux sorties, une par appareil de mesure, la sortie vers l'appareil de mesure de composition gazeuse comportant une restriction de passage réduisant la section de passage par rapport à l'autre sortie,
- le dispositif de connexion du cathéter au dispositif d'assistance respiratoire est configuré de manière à permettre la communication aérique entre le canal principal et l'environnement extérieur/l'air ambiant afin que l'ensemble dispositif d'assistance respiratoire + cathéter soit ou reste un système ouvert,
- le cathéter est tubulaire et la lumière du cathéter à un diamètre interne d'au moins 2 mm,
- le raccord de liaison gazeuse comporte un robinet trois voies, une première des voies étant du côté de la lumière du cathéter, une seconde des voies étant du côté l'appareil de mesure de pression et une troisième des voies étant du côté l'appareil de mesure de composition gazeuse, une restriction de passage étant présente entre la troisième voie et l'appareil de mesure de composition gazeuse en sortie du robinet afin que la liaison gazeuse vers l'appareil de mesure de composition gazeuse présente une section de passage plus réduite que la section de passage de la liaison gazeuse vers l'appareil de mesure de pression,
- le robinet trois voies comporte un organe de commande permettant, selon la position dudit organe de commande, au moins : la fermeture de la lumière du cathéter ou, alors, la liaison gazeuse entre la lumière du cathéter et l'appareil de mesure de composition gazeuse ou, alors, la liaison gazeuse entre la lumière du cathéter et l'appareil de mesure de pression ou, alors, la liaison gazeuse entre la lumière du cathéter et les appareils de mesure de composition gazeuse et de mesure de pression,
- le dispositif d'assistance respiratoire auquel est destiné le cathéter est choisi parmi un masque facial, une sonde d'intubation trachéale ou une sonde à masque pharyngé ou laryngé,
- le cathéter comporte en outre en parallèle au moins un canal d'injection d'oxygène étendu entre l'extrémité proximale du cathéter où ledit canal peut être relié à une source d'oxygène sous pression et une sortie d'oxygène débouchant en périphérie du cathéter et en retrait de l'extrémité distale du cathéter, le canal d'injection et la lumière étant indépendants (sans communication) l'un de l'autre, ledit cathéter à canal d'injection d'oxygène étant notamment destiné à être utilisé dans un dispositif d'assistance respiratoire ne comportant pas de moyens d'injection d'oxygène dans son canal principal,
- ledit au moins un canal d'injection d'oxygène est disposé dans l'épaisseur de la paroi du cathéter,
- ledit au moins un canal d'injection d'oxygène est un canal coaxial externe au cathéter,
- ledit canal coaxial externe au cathéter comporte une paroi externe rigide, le cathéter étant souple pour être courbé,
- ledit canal coaxial externe au cathéter comporte une paroi externe souple et se gonflant sous la pression de l'oxygène injecté y passant,
- ledit au moins un canal d'injection d'oxygène est au moins une tubulure externe et parallèle au cathéter et solidaire de ce dernier,
- le canal d'injection d'oxygène est configuré de manière à ce que sa sortie d'oxygène soit dans le canal principal, vers l'extrémité distale du dispositif d'assistance respiratoire,
- la sortie d'oxygène du canal d'injection d'oxygène comporte au moins un orifice créant un jet incliné par rapport à la paroi externe du cathéter et qui est orienté pour venir frapper la paroi interne du canal principal du dispositif d'assistance respiratoire,
- l'orientation du/des jets inclinés sortant de la paroi externe du cathéter est radiale, perpendiculaire à ladite paroi externe,
- l'orientation du/des jets inclinés sortant de la paroi externe du cathéter est distale (jets orientés vers les poumons) avec un angle d'inclinaison par rapport à ladite paroi externe compris entre 45° et 90°,
- l'orientation du/des jets inclinés sortant de la paroi externe du cathéter est proximale (jets orientés vers l'extérieur) avec un angle d'inclinaison par rapport à ladite paroi externe compris entre 45° et 90°,
- le cathéter comporte deux groupes d'orifices de jets inclinés, un premier groupe d'au moins un orifice orientant proximalement (jets orientés vers l'extérieur) le/les jets inclinés sortant de la paroi externe du cathéter et un second groupe d'au moins un orifice orientant distalement (jets orientés vers les poumons) le/les jets inclinés sortant de la paroi externe du cathéter, un détecteur de compression-relâchement thoracique commandant un inverseur disposé sur l'alimentation continue en oxygène pour envoyer l'oxygène alternativement vers le premier groupe lors de la compression thoracique et vers le second groupe lors du relâchement thoracique,
- le cathéter comporte un ensemble d'orifices de jets inclinés répartis sur le pourtour du cathéter,
- l'appareil de mesure de composition gazeuse est amovible,
- l'appareil de mesure de pression gazeuse est amovible,
- l'appareil de mesure de pression gazeuse est inamovible sur le cathéter,
- le cathéter est à usage unique,
- l'appareil de mesure de composition gazeuse est amovible pour être réutilisé sur plusieurs cathéters,
- l'appareil de mesure de pression gazeuse est à usage unique.

L'invention propose également un ensemble dispositif d'assistance respiratoire et cathéter, le cathéter étant un cathéter de mesures mixtes tel que présenté et le cathéter étant amovible du dispositif d'assistance respiratoire.

### DESCRIPTION DETAILLEE D'UN EXEMPLE DE RÉALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 représente une vue en coupe longitudinale d'un ensemble sonde d'intubation à injection d'oxygène par jets défléchis et cathéter selon l'invention,
- la figure 2 représente une vue en coupe longitudinale de l'extrémité distale d'un ensemble sonde d'intubation sans moyens d'injection d'oxygène et cathéter à injection d'oxygène selon une variante de l'invention, et
- les figures 3A et 3B représentent des coupes transversales d'un cathéter à injection d'oxygène dans deux modes de réalisation.

### Dispositif

Le cathéter de l'invention est destiné à être introduit dans un dispositif d'assistance respiratoire qui est une sonde d'intubation et il est destiné à des appareils de mesure passifs, c'est-à-dire que ces appareils de mesure n'ont pas une fonction d'aspiration des gaz. La circulation des gaz dans la lumière du cathéter est donc passive. En effet, étant donné que le cathéter sert à la fois pour des mesures de pression, ou à tout le moins d'indication d'une circulation correcte des gaz dans le dispositif d'assistance, et de composition des gaz, en particulier du CO₂, une aspiration forcée par l'appareil de mesure de composition des gaz fausserait les mesures de pression. Pour cette même raison, la lumière du cathéter ne peut pas non plus servir à l'injection de gaz ou autres fluides à destination de la personne intubée.

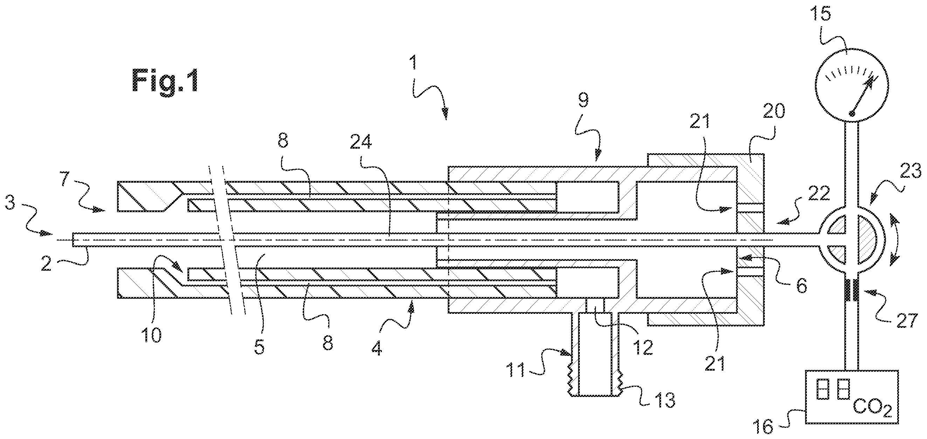

On va décrire dans le détail et en relation avec la figure 1 un exemple d'application sur une sonde d'intubation trachéale du type de celle présentée dans le document FR2010/03343 (FR 2 963 740 A1). Cette sonde 4 présente l'avantage d'assurer une injection de gaz respiratoire, oxygène en pratique, par des jets défléchis vers l'intérieur du canal principal 5 de la sonde et vers son extrémité distale 7, la sonde étant un système ouvert à son extrémité proximale 6. Il en résulte la création d'une soupape virtuelle du fait de la déflexion du gaz injecté vers le centre du canal principal 5 dans ce système ouvert et qui présente en plus un espace mort réduit du fait que cette injection de gaz est distale. Des canaux auxiliaires 8 réalisés dans et le long de la paroi de la sonde 4 permettent d'envoyer l'oxygène vers des orifices de sortie à moyens de déflexion 10 permettant de créer les jets défléchis. Une ligne d'oxygène sous pression est connectée à un mamelon 11 à vissage 13 disposé sur un embout proximal 9 de la sonde 4 d'intubation. Dans l'embout 9, une chambre circulaire permet de relier le mamelon 11 par l'intermédiaire d'un passage 12 aux canaux auxiliaires 8 et donc d'envoyer de l'oxygène sous pression dans lesdits canaux auxiliaires 8. Les canaux auxiliaires 8 et leur alimentation en oxygène forment un circuit fluidique indépendant/isolé du canal principal 5 de la sonde 4, les deux se rejoignant/communiquant seulement au niveau des orifices de sortie à moyens de déflexion 10.

L'injection d'oxygène est de préférence continue lors des tentatives de réanimation cardiopulmonaire avec massage thoracique/cardiaque continu de la personne réanimée et intubée par la sonde 4.

On comprend que si on effectue des mesures directement côté extrémité proximale, ces mesures seront faussées, au moins pour ce qui concerne la composition des gaz expirés par la personne, du fait qu'ils auront traversés la zone d'injection d'oxygène.

On introduit donc le cathéter 2 de l'invention dans la sonde 4 d'intubation installée dans la personne pour prélever les gaz côté distal de l'injection des gaz respiratoires par les jets défléchis. Cette introduction ne présente pas de difficulté car la sonde 4 d'intubation, qui est à injection d'oxygène par jets défléchis en distal 7, est ouverte à son extrémité proximale 6, à l'extérieur de la personne. On obtient un ensemble 1 formé d'un appareil d'assistance respiratoire 4 et d'un cathéter 2 amovible. Ce cathéter 2 présente une extrémité distale 3 qui est insérée du côté de la personne intubée et une extrémité proximale 22 à l'extérieur de la personne. Un dispositif de connexion 20 est prévu à l'extrémité proximale 22 du cathéter 2 pour connexion amovible à l'extrémité proximale 6 de la sonde 4 d'intubation. Ce dispositif de connexion 20 du cathéter est configuré pour assurer un maintien physique/de retenue entre la sonde 4 et le cathéter 2 tout en laissant le système ouvert, c'est-à-dire que le canal principal 5 de la sonde d'intubation reste ouvert sur l'extérieur à l'extrémité proximale 6 de la sonde. Dans l'exemple représenté, cette ouverture du système est conservée par la mise en œuvre d'orifices de passage 21 à travers le dispositif de connexion 20. Toujours dans cet exemple, le dispositif de connexion 20 vient encapuchonner l'embout 9 à son extrémité proximale 6 tout en conservant un système ouvert pour la sonde 4 mais dans d'autres modalités de réalisation, le dispositif de connexion 20 s'insère dans l'extrémité proximale 6 de l'embout 9.

Le cathéter 2 est amovible et le dispositif de connexion du cathéter peut être retiré de l'extrémité proximale 6 de la sonde d'intubation afin d'extraire le cathéter hors de la sonde. On pourra vérifier que la sonde d'intubation trachéale du document FR2010/03343 (FR 2 963 740 A1) est particulièrement utile dans le contexte de la réanimation cardiopulmonaire car une fois la personne « repartie » (son cœur recommence à battre), on peut brancher sur l'extrémité proximale de la sonde un respirateur, bien évidemment après avoir retiré le cathéter hors de la sonde. Une fois le respirateur branché, le système devient fermé et l'oxygène ne doit plus être injecté par les jets défléchis dans la sonde, ce que la sonde en question permet de contrôler automatiquement du fait de la présence d'un moyen détrompeur empêchant des branchements erronés et/ou simultanés sur la sonde entre l'injection d'oxygène et le respirateur.

En ce qui concerne de nouveau le cathéter 2, un raccord de liaison gazeuse entre la lumière 24 du cathéter (son canal interne) et des appareils de mesure 15, 16 est également prévu à l'extrémité proximale 22 du cathéter. Ces appareils de mesure sont un appareil de mesure de composition gazeuse 16, plus particulièrement de CO₂, et un appareil de mesure de pression gazeuse 15, qui est typiquement un simple indicateur de pression de type manomètre ou même de débit gazeux. Le terme manomètre utilisé ici doit être pris dans le sens général d'un moyen d'indication que la circulation des gaz dans le dispositif d'assistance respiratoire s'effectue ou pas dans des conditions satisfaisantes et pas forcément une mesure précise de pression.

En effet, dans des conditions d'urgence, un indicateur simple de la bonne circulation des gaz entre la personne, l'environnement et l'injection d'oxygène suffit. Par exemple un repère pouvant se déplacer dans un tube peut indiquer un fonctionnement normal s'il oscille avec une amplitude importante, une fuite s'il n'oscille pas ou peu vers les pressions basses ou une occlusion s'il reste à haute pression. Les résultats des mesures/indications des appareils de mesure peuvent également permettre de vérifier le bon positionnement et/ou l'étanchéité de la sonde dans la trachée par rapport à l'œsophage ou, alors, en laryngé ou pharyngé pour les deux derniers types correspondants de sondes autres que trachéale.

Ce raccord de liaison gazeuse comporte un robinet 23 trois voies avec la première des voies qui est du côté de la lumière 24 du cathéter, la seconde des voies du côté l'appareil de mesure de pression 15 et la troisième des voies du côté l'appareil de mesure de composition gazeuse 16. Une restriction 27 de passage est disposée sur la troisième voie entre le robinet 23 et l'appareil de mesure de composition gazeuse 16. Grâce à cette restriction 27, la mesure ou à tout le moins l'indication fournie par l'appareil de mesure de pression est très peu perturbée. En outre, grâce au robinet 23 trois voies, si l'on met en œuvre un manomètre relativement précis et que l'on souhaite une mesure relativement précise on peut tourner la commande du robinet pour n'avoir une pleine communication qu'entre la lumière 24 du cathéter et le manomètre 15 afin de supprimer la fuite, même minime du fait de la restriction, créée par l'appareil de mesure de composition gazeuse 16.

Dans une variante de réalisation, on peut prévoir une communication commandée à l'ouverture et revenant automatiquement à la fermeture à l'arrêt de la commande (type bouton poussoir) entre la lumière 24 de la partie proximale du cathéter et la source d'oxygène sous pression afin de purger de temps à autre le cathéter des sécrétions ou autres ayant pu s'y engager. Toutefois, cela ajoute une complexité de réalisation et d'utilisation qui n'est pas forcément nécessaire dans certaines conditions d'urgence.

Sur la figure 1, l'extrémité distale 3 du cathéter 2 en place dans la sonde va au-delà de l'extrémité distale 7 de la sonde 4, ce qui est préférable pour des mesures relativement précises. Toutefois, on peut prévoir que l'extrémité distale du cathéter en place soit dans le canal principal 5 de la sonde mais dans ce cas ce sera du côté distal/de la personne par rapport aux jets défléchis d'oxygène injecté pour éviter les inconvénients des mesures classiques en proximal.

Le cathéter est donc adapté, notamment en ce qui concerne sa longueur et son dispositif de connexion, au dispositif d'assistance respiratoire sur lequel il est utilisé. Dans une variante de réalisation, le tube du cathéter peut coulisser à travers le dispositif de connexion 20 tout en étant suffisamment retenu par ce dernier en l'absence de traction ou de poussée conséquente sur le tube du cathéter. Il est alors possible de régler la position de l'extrémité distale 3 du cathéter par rapport à l'extrémité distale 7 du dispositif d'assistance respiratoire en tirant ou poussant le tube du cathéter. Le tube du cathéter peut comporter sur sa surface externe des repères visibles correspondant au type du dispositif d'assistance respiratoire sur lequel il est utilisé : l'utilisateur positionnera en conséquence le tube du cathéter par rapport au dispositif de connexion 20 pour que l'extrémité distale du cathéter soit positionnée par rapport à l'extrémité distale du dispositif d'assistance respiratoire dans une position interne ou externe au canal principal déterminée. On peut éventuellement prévoir un moyen de serrage entre le dispositif de connexion 20 et le tube du cathéter qui sera serré une fois la position souhaitée atteinte.

A noter que sur la figure 1, pour des raisons de simplification, on n'a pas représenté le ballonnet d'étanchéité qui entoure la sonde côté distal et qui est destiné à assurer l'étanchéité entre la sonde et la trachée dans ce cas particulier. Ce ballonnet est gonflé de l'extérieur par l'intermédiaire d'un microcanal de gonflage (non représenté mais similaire à un canal auxiliaire 8) allongé le long et dans la paroi de la sonde et qui est parallèle aux canaux auxiliaires qui servent eux à l'injection d'oxygène. Les canaux auxiliaires 8 et le microcanal de gonflage sont indépendants/isolés entre eux. Dans une variante, un ballonnet double est mis en œuvre, l'un dans l'autre, l'un des deux étant gonflé par la pression régnant localement dans ou autour de la sonde afin de créer des variations dans la pression de serrage contre la paroi de la trachée et soulager quelque peu cette dernière.

A la base, le cathéter selon l'invention comporte le tube du cathéter définissant la lumière 24 de communication entre l'extrémité distale et proximale, le dispositif de connexion 20 et le raccord de liaison gazeuse 23. Dans une variante, il peut être fourni avec en plus l'appareil de mesure de pression 15 gazeuse. Il est aussi possible de fournir l'ensemble cathéter + dispositif d'assistance respiratoire. De préférence, l'appareil de mesure de composition gazeuse est un appareil réutilisable et qui sera connecté au cathéter lors de la réanimation.

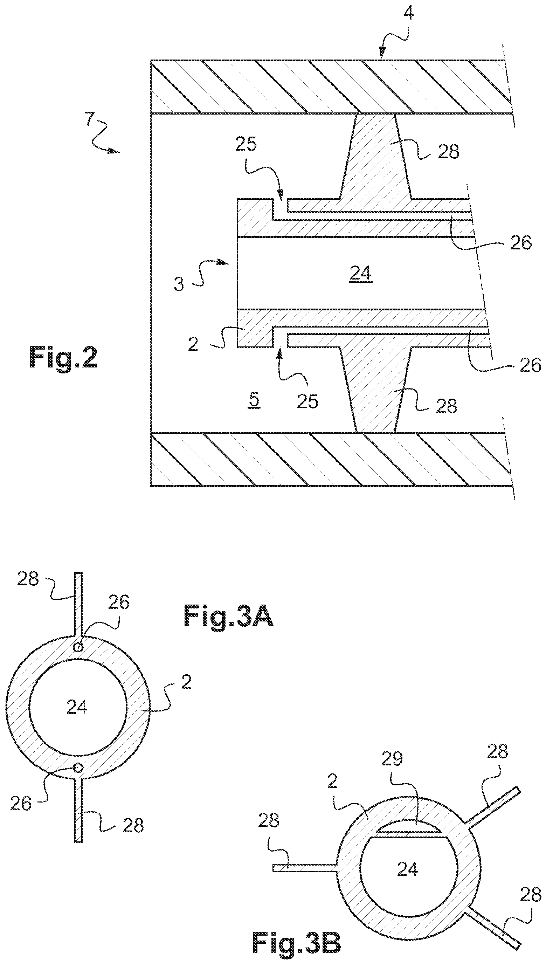

Sur la figure 2, une variante de la sonde 2 permet de créer une injection distale d'oxygène, donc à espace mort réduit, dans une sonde 4 d'intubation qui ne comporte pas de moyen propre/spécifique d'injection d'oxygène en distal ou, alors, qu'on ne souhaite pas utiliser. Comme précédemment, le ballonnet d'étanchéité distal n'a pas été représenté. Pour cela, le cathéter 2 comporte en plus un moyen d'injection d'oxygène (ou plus généralement de gaz respiratoire) qui est délivré à la personne par des jets défléchis latéralement, en périphérie de la paroi externe du cathéter, vers son extrémité distale 3. Dans l'exemple représenté, les jets créés au niveau d'orifices de sortie 25 latéraux sont radiaux et viennent frapper la paroi interne de la sonde d'intubation 4. De préférence, on met en œuvre au moins trois jets dont les orifices de sortie sont répartis équiangulairement autour du cathéter 2. Du fait de cette répartition régulière des orifices de sortie des jets défléchis, le cathéter aura tendance à se placer sensiblement au centre du canal principal de la sonde d'intubation. Il est toutefois préférable de mettre en œuvre des moyens de stabilisation de la position du cathéter dans le canal principal de la sonde afin d'éviter un éventuel battement de l'extrémité distale du cathéter du fait du ou des jets latéraux. Ces moyens de stabilisation sont ici de fines ailettes 28 ou tout autre moyen écarteur solidaire du cathéter et réduisant le moins possible la section du canal principal 5. Les ailettes 28 qui sont au nombre d'au moins deux et répartis radialement équiangulairement autour du cathéter, peuvent présenter une certaine souplesse afin de faciliter l'introduction du cathéter 2 dans la sonde 4.

On a représenté des canalicules 26 d'injection d'oxygène dans l'épaisseur et le long de la paroi du cathéter 2 mais on envisage le jumelage d'un cathéter et d'une tubulure solidaire parallèle et externe au cathéter, tubulure qui est destinée à l'injection d'oxygène. Ainsi, figure 3A on a représenté une coupe transversale du cathéter de la figure 2 au niveau de ses ailettes 28. Il est également possible de prévoir une division en deux de la lumière du cathéter sur sa longueur pour réserver une partie de section réduite pour l'injection d'oxygène sous pression, ces deux parties ne communiquant pas entre elles. Ainsi, figure 3B on a représenté une coupe transversale d'un cathéter 2 ayant une partie 29 de sa lumière 24 réservée à l'injection d'oxygène et qui est de section plus réduite que le reste de la lumière 24.

De préférence, la configuration du cathéter à injection d'oxygène est telle que son extrémité distale, une fois qu'il est en place dans la sonde d'intubation, se termine dans le canal principal 5 de la sonde. En effet, il est préférable que les jets d'oxygène viennent frapper la paroi interne de la sonde plutôt que la paroi de la trachée. Ainsi, de préférence, même si l'extrémité distale 3 du cathéter 2 du fait de sa configuration, va au-delà de l'extrémité distale 7 du dispositif d'assistance respiratoire 4, les orifices de sortie 25 latéraux restent dans le canal principal 5 et ne sont pas positionnés au-delà de l'extrémité distale 7 du dispositif d'assistance respiratoire.

L'oxygène est envoyé dans les canalicules 26 d'injection d'oxygène par un raccord (non représenté) côté extrémité proximale 22 du cathéter 2, par exemple disposé sur le dispositif de connexion 20 du cathéter, tout en permettant de conserver un système ouvert pour la sonde et une étanchéité entre la lumière du cathéter et l'oxygène.

On comprend que toute autre disposition, notamment en nombre de canalicules ou tubulures, orifices de sortie, configuration, taille, est possible pour parvenir au même résultat d'injection en distal d'oxygène par le cathéter de mesures mixtes, de pression et de composition gazeuse.

### Procédé

Classiquement, sur une personne en arrêt cardiorespiratoire, les premiers secours, après avoir dégagé les voies respiratoires, procèdent à une intubation avec une sonde 4 d'intubation, typiquement trachéale ou à masque pharyngé ou laryngé selon les cas. Les moyens d'étanchéité à type de ballonnet de la sonde sont gonflés et on s'assure du bon positionnement de l'extrémité distale de la sonde. La sonde d'intubation peut être simple, c'est-à-dire simplement assurer une communication entre les voies respiratoires et l'extérieur, l'oxygène étant délivré à l'extrémité proximale de la sonde par exemple avec un ballon, ou alors, être du type à injection distale d'oxygène par des jets défléchis d'un système ouvert du type de celui de Monsieur BOUSSIGNAC, ce dernier système pouvant simplifier la réanimation du fait que l'injection d'oxygène peut être continue. On peut noter que la mise en œuvre d'un cathéter à injection d'oxygène permet de faire bénéficier à une sonde d'intubation simple, certains des avantages des systèmes ouverts de Monsieur BOUSSIGNAC. Une fois la sonde d'intubation en place, on peut installer le cathéter 2 de mesures mixtes, ce qui se fait simplement dans un système ouvert. La surveillance des mesures peut permettre de détecter initialement un mauvais positionnement de la sonde, un défaut d'étanchéité ou toute autre anomalie et, ultérieurement, leur survenue. Une fois la personne « repartie », on retire le cathéter pour mettre en œuvre des moyens de ventilation adaptés, manuels ou mécaniques.

On comprend que l'invention qui vient d'être décrite en relation avec une sonde d'intubation trachéale peut en pratique s'appliquer à des sondes d'intubation autres et notamment à masque laryngé ou pharyngé ou même, dérivés des principes décrits jusqu'à présent, à un masque facial. Dans ce dernier cas, la coque du masque facial et ses éventuels moyens d'injection d'oxygène à jets défléchis d'un système ouvert s'apparente au tube de la sonde décrite précédemment. Le cathéter d'une longueur réduite pour se terminer dans le masque, à l'extérieur de la personne, pourra être passé dans l'orifice principal du masque sur lequel est connecté un dispositif d'injection d'oxygène de type système ouvert et donc en traversant ce dernier. Un exemple de tel masque à dispositif d'injection d'oxygène est représenté dans le document FR 2 911 073 A1.

Dans une variante du masque facial, un orifice supplémentaire, spécifique, de mesure est réalisé à travers la coque du masque et le cathéter y est fixé et, dans ce cas, le cathéter de mesures mixtes ne passe pas par le passage des gaz inspirés et expirés contrairement aux exemples précédents. A noter que le cathéter de mesures mixtes peut également comporter un moyen d'injection d'oxygène dans ces applications à un masque facial.

## Revendications

1. Cathéter (2) de mesures mixtes destiné à être introduit et passé dans un canal principal (5) interne de circulation de gaz respiratoires d'un dispositif d'assistance respiratoire (4), ledit dispositif d'assistance respiratoire (4) comportant une extrémité distale (7) destinée à être placée sur ou introduite dans les voies respiratoires d'une personne et une extrémité proximale (6) destinée à être disposée à l'extérieur de la personne et opposée à l'extrémité distale (7), ledit cathéter (2) ayant une extrémité proximale (22) et une extrémité distale (3) entre lesquelles s'étend intérieurement une lumière (24) dudit cathéter (2), ledit cathéter (2) comportant à son extrémité proximale, d'une part, un dispositif de connexion (20) à l'extrémité proximale du dispositif d'assistance respiratoire et, d'autre part, une liaison gazeuse de la lumière (24) du cathéter (2) à un appareil de mesure, le cathéter (2) étant configuré pour que, une fois introduit dans le canal principal (5) et connecté au dispositif d'assistance respiratoire, son extrémité distale (3) se positionne vers l'extrémité distale (7) du dispositif d'assistance respiratoire (4) ou, à tout le moins, du côté distal d'un/de jets d'oxygène injecté (10) dans le canal principal (5) du dispositif d'assistance respiratoire (4) dans le cas où le dispositif d'assistance respiratoire (4) comporterait des moyens d'injection (10, 8) d'oxygène dans son canal principal (5)
**caractérisé en ce que** ledit cathéter (2) comporte à son extrémité proximale (22) un raccord de liaison gazeuse (23) de la lumière (24) du cathéter (2) à un appareil de mesure de composition gazeuse (16) et à un appareil de mesure de pression (15) gazeuse, et **en ce que** dans le raccord de liaison gazeuse (23), la liaison gazeuse pour l'appareil de mesure de composition gazeuse (16) présente une section de passage plus réduite (27) que la section de passage de la liaison gazeuse pour l'appareil de mesure de pression (15).

2. Cathéter (2) selon la revendication 1, **caractérisé en ce qu'**il est tubulaire et que la lumière (24) du cathéter (2) à un diamètre interne d'au moins 2 mm.

3. Cathéter (2) selon la revendication 1 ou 2, **caractérisé en ce que** le raccord de liaison gazeuse comporte un robinet trois voies (23), une première des voies étant du côté de la lumière (24) du cathéter (2), une seconde des voies étant du côté l'appareil de mesure de pression (15) et une troisième des voies étant du côté l'appareil de mesure de composition gazeuse (16), une restriction de passage (27) étant présente entre la troisième voie et l'appareil de mesure de composition gazeuse (16) en sortie du robinet afin que la liaison gazeuse vers l'appareil de mesure de composition gazeuse (16) présente une section de passage plus réduite que la section de passage de la liaison gazeuse vers l'appareil de mesure de pression (15).

4. Cathéter (2) selon la revendication 3, **caractérisé en ce que** le robinet trois voies (23) comporte un organe de commande permettant, selon la position dudit organe de commande, au moins : la fermeture de la lumière (24) du cathéter (2) ou la liaison gazeuse entre la lumière (24) du cathéter et l'appareil de mesure de composition gazeuse ou la liaison gazeuse entre la lumière (24) du cathéter et l'appareil de mesure de pression ou la liaison gazeuse entre la lumière (24) du cathéter et les appareils de mesure de composition gazeuse et de mesure de pression.

5. Cathéter (2) selon l'une quelconque des revendications précédentes, **caractérisé en ce qu'**il comporte en outre en parallèle au moins un canal d'injection (26) d'oxygène étendu entre l'extrémité proximale (22) du cathéter où ledit canal peut être relié à une source d'oxygène sous pression et une sortie (25) d'oxygène débouchant en périphérie du cathéter (2) et en retrait de l'extrémité distale (3) du cathéter, le canal d'injection (26) et la lumière (24) étant indépendants l'un de l'autre, ledit cathéter (2) à canal d'injection (26) d'oxygène étant notamment destiné à être utilisé dans un dispositif d'assistance respiratoire (4) ne comportant pas de moyens d'injection d'oxygène dans son canal principal (5).

6. Cathéter (2) selon la revendication 5, **caractérisé en ce que** le canal d'injection (26) d'oxygène est configuré de manière à ce que sa sortie (25) d'oxygène soit dans le canal principal (5), vers l'extrémité distale (7) du dispositif d'assistance respiratoire (4).

7. Cathéter (2) selon la revendication 6, **caractérisé en ce que** la sortie d'oxygène du canal d'injection (26) d'oxygène comporte au moins un orifice (25) créant un jet incliné par rapport à la paroi externe du cathéter (2) et qui soit donc orienté pour venir frapper la paroi du canal principal (5) du dispositif d'assistance respiratoire (4).

8. Cathéter (2) selon la revendication 7, **caractérisé en ce qu'**il comporte un ensemble d'orifices (25) de jets inclinés répartis sur le pourtour du cathéter (2).

9. Ensemble (1) dispositif d'assistance respiratoire (4) et cathéter (2), **caractérisé en ce que** le cathéter est un cathéter (2) de mesures mixtes selon l'une quelconque des revendications précédentes et **en ce qu'**il est amovible du dispositif d'assistance respiratoire.

10. Ensemble (1) selon la revendication 9, **caractérisé en ce que** le dispositif d'assistance respiratoire (4) est choisi parmi un masque facial, une sonde d'intubation trachéale (4) ou une sonde à masque pharyngé ou laryngé.

11. Ensemble (1) selon la revendication 9 ou la revendication 10, **caractérisé en ce que** le cathéter (2) est à usage unique et qu'il est destiné à être raccordé à un appareil de mesure de composition gazeuse et à un appareil de mesure de pression gazeuse et **en ce que** l'appareil de mesure de composition gazeuse (16) est amovible pour être réutilisé sur plusieurs cathéters et l'appareil de mesure de pression (15) est à usage unique.

## Patentansprüche

1. Katheter (2) für kombinierte Messungen, der dazu ausgelegt ist, in einen inneren Hauptkanal (5) für den Durchfluß von Atemgasen einer Beatmungshilfevorrichtung (4) eingeführt und in diesem bewegt zu werden, wobei die Beatmungshilfevorrichtung (4) ein distales Ende (7), das dazu bestimmt ist, auf die Atemwege einer Person aufgesetzt oder in diese eingeführt zu werden, und ein proximales Ende (6), das dazu bestimmt ist, außerhalb der Person angeordnet zu werden und das dem distalen Ende (7) entgegengesetzt ist, aufweist, wobei der Katheter (2) ein proximales Ende (22) und ein distales Ende (3) aufweist, zwischen denen sich ein innerer Kanal (24) des Katheters (2) erstreckt, wobei der Katheter (2) an seinem proximalen Ende einerseits eine Vorrichtung (20) für den Anschluß an das proximale Ende der Beatmungshilfevorrichtung und andererseits eine Gasverbindung des Kanals (24) des Katheters (2) mit einem Meßgerät aufweist, wobei der Katheter dazu ausgelegt ist, daß sein distales Ende (3), wenn der Katheter in den Hauptkanal (5) eingeführt und mit der Beatmungshilfevorrichtung (4) verbunden ist, zum distalen Ende (7) der Beatmungshilfevorrichtung hin oder, wenn die Beatmungshilfevorrichtung (4) Mittel (10, 8) zum Einspritzen von Sauerstoff in den Hauptkanal (5) aufweist, wenigstens auf der distalen Seite eines Strahls oder von Strahlen eingespritzten Sauerstoffs (10) positioniert ist,
**dadurch gekennzeichnet, daß** der Katheter (2) an seinem proximalen Ende (22) einen Anschluß (23) für eine Gasverbindung des Kanals (24) des Katheters (2) mit einem Gerät (16) zum Messen der Zusammensetzung des Gases und mit einem Gerät (15) zum Messen des Gasdrucks aufweist und daß die Gasverbindung für das Gerät (16) zum Messen der Zusammensetzung des Gases im Anschluß (23) für eine Gasverbindung einen Durchlaß (27) mit einem kleineren Querschnitt als dem Querschnitt der Gasverbindung für das Druckmeßgerät (15) aufweist.

2. Katheter (2) gemäß Anspruch 1, **dadurch gekennzeichnet, daß** er rohrförmig ist und daß der Kanal (24) des Katheters (2) einen inneren Durchmesser von wenigstens 2 mm aufweist.

3. Katheter (2) gemäß Anspruch 1 oder 2, **dadurch gekennzeichnet, daß** der Anschluß für eine Gasverbindung ein Dreiwegeventil (23) aufweist, wobei ein erster der Wege auf der Seite des Kanals (24) des Katheters (2) ist, ein zweiter der Wege auf der Seite des Druckmeßgeräts (15) ist und ein dritter der Wege auf der Seite (16) zum Messen der Zusammensetzung der Gase ist, wobei eine Verringerung (27) des Durchgangs zwischen dem dritten Weg und dem Gerät (16) zum Messen der Zusammensetzung der Gase am Ausgang des Ventils vorliegt, damit die Gasverbindung zum Gerät (16) zum Messen der Zusammensetzung der Gase einen kleineren Durchflußquerschnitt als den Durchflußquerschnitt der Gasverbindung zum Druckmeßgerät (15) aufweist.

4. Katheter (2) gemäß Anspruch 3, **dadurch gekennzeichnet, daß** das Dreiwegeventil (23) ein Steuerorgan aufweist, das je nach Stellung des Steuerorgans wenigstens ermöglicht: Sperren des Kanals (24) des Katheters (2) oder Gasverbindung zwischen dem Kanal (24) des Katheters und dem Gerät zum Messen der Zusammensetzung der Gase oder Gasverbindung zwischen dem Kanal (24) des Katheters und dem Druckmeßgerät oder Gasverbindung zwischen dem Kanal (24) und den Geräten zum Messen der Zusammensetzung der Gase bzw. des Drucks.

5. Katheter (2) gemäß einem der vorangehenden Ansprüche, **dadurch gekennzeichnet, daß** er außerdem wenigstens einen parallelen Kanal (26) zum Einspritzen von Sauerstoff aufweist, der sich zwischen dem proximalen Ende (22) des Katheters (2), an dem der Kanal mit einer unter Druck stehenden Sauerstoffquelle verbunden werden kann, und einem Sauerstoffausgang (25), der am Umfang des Katheters (2) und gegenüber dem distalen Ende (3) zurückgezogen mündet, erstreckt, wobei der Einspritzkanal (26) und der Kanal (24) voneinander unabhängig sind, wobei der Katheter (2) mit Sauerstoffeinspritzkanal (26) insbesondere dazu bestimmt ist, in einer Beatmungshilfevorrichtung (4) verwendet zu werden, die keine Mittel zum Einspritzen von Sauerstoff in ihren Hauptkanal (5) aufweist

6. Katheter (2) gemäß Anspruch 5, **dadurch gekennzeichnet, daß** der Kanal (26) zum Einspritzen von Sauerstoff derart ausgelegt ist, daß sich dessen Sauerstoffausgang (25) im Hauptkanal (5) zum distalen Ende (7) der Beatmungshilfevorrichtung (4) hin befindet.

7. Katheter (2) gemäß Anspruch 6, **dadurch gekennzeichnet, daß** der Sauerstoffausgang des Kanals (26) zum Einspritzen von Sauerstoff wenigstens ein Loch (25) aufweist, das einen gegenüber der Außenwand des Katheters (2) geneigten Strahl erzeugt, der somit so orientiert ist, daß er auf die Wand des Hauptkanals (5) der Beatmungshilfevorrichtung 4) trifft.

8. Katheter (2) gemäß Anspruch 7, **dadurch gekennzeichnet, daß** er eine Anzahl auf dem Umfang des Katheters (2) verteilter Löcher (25) mit schrägen Strahlen aufweist.

9. Gesamtheit (1) aus einer Beatmungshilfevorrichtung (4) und einem Katheter (2), **dadurch gekennzeichnet, daß** der Katheter ein Katheter (2) für gemischte Messungen gemäß einem der vorangehenden Ansprüche ist und daß er von der Beatmungshilfevorrichtung abnehmbar ist.

10. Gesamtheit (1) gemäß Anspruch 9, **dadurch gekennzeichnet, daß** die Beatmungshilfevorrichtung (4) aus einer Gesichtsmaske, einer trachealen Intubationssonde (4) oder einer Sonde mit Rachenraum- oder Kehlkopfmaske ausgewählt ist.

11. Gesamtheit (1) gemäß Anspruch 9 oder Anspruch 10, **dadurch gekennzeichnet, daß** der Katheter ein Einwegkatheter ist und daß er dazu bestimmt ist, mit einem Gerät zum Messen der Zusammensetzung der Gase und mit einem Gerät zum Messen des Gasdrucks verbunden zu werden, und daß das Gerät (16) zum Messen der Zusammensetzung der Gase abnehmbar ist, um mit mehreren Kathetern wiederverwendet zu werden, und daß das Druckmeßgerät (15) ein Einweggerät ist.

## Claims

1. A combined measurement catheter (2) intended to be introduced into and passed through a main inner respiratory gas channel (5) of a breathing assistance device (4), said breathing assistance device (4) including a distal end (7) intended to be placed on or introduced into the airways of a person and a proximal end (6) intended to be arranged outside of the person and opposite to the distal end (7), said catheter (2) having a proximal end (22) and a distal end (23) between which extends internally a lumen (24) of said catheter (2), said catheter (2) having at its proximal end, on the one hand, a device (20) for connection to the proximal end of the breathing assistance device and, on the other hand, a gaseous connection of the lumen (24) of the catheter (2) to a measurement device, the catheter (2) being configured so that, once introduced into the main channel (5) and connected to the breathing assistance device, its distal end (3) is positioned towards the distal end (7) of the breathing assistance device (4) or, at least, on the distal side of one/several injected oxygen jets (10) in the main channel (5) of the breathing assistance device (4) in the case where the breathing assistance device (4) would include oxygen injection means (10, 8) in its main channel (5),
**characterized in that** said catheter (2) includes, at its proximal end (22), a connector (23) for gaseous connection of the lumen (24) of the catheter (2) to a gaseous composition measurement apparatus (16) and to a gaseous pressure measurement apparatus (15), and **in that**, in the connector (23) for gaseous connection, the passage cross-section (27) of the gaseous connection for the gaseous composition measurement apparatus (16) is smaller than the passage cross-section of the gaseous connection for the pressure measurement apparatus (15).

2. The catheter (2) according to claim 1, **characterized in that** it is tubular and **in that** the lumen (24) of the catheter (2) has an inner diameter of less than 2 mm.

3. The catheter (2) according to claim 1 or 2, **characterized in that** the connector for gaseous connection includes a three-way valve (23), a first one of the ways being on the side of the lumen (24) of the catheter (2), a second one of the ways being on the side of the pressure measurement apparatus (15) and a third one of the ways being on the side of the gaseous composition measurement apparatus (16), a passage narrowing (27) being present between the third way and the gaseous composition measurement apparatus (16) at the outlet of the valve so that the gaseous connection to the gaseous composition measurement apparatus (16) has a smaller passage cross-section than the passage cross-section of the gaseous connection to the pressure measurement apparatus (15).

4. The catheter (2) according to claim 3, **characterized in that** the three-way valve (23) includes a control member for, according to the position of said control member, at least: closing the lumen (24) of the catheter (2) or the gaseous connection between the lumen (24) of the catheter and the gaseous composition measurement apparatus or the gaseous connection between the lumen (24) of the catheter and the pressure measurement apparatus or the gaseous connection between the lumen (24) of the catheter and the gaseous composition measurement and pressure measurement apparatuses.

5. The catheter (2) according to any one of the preceding claims, **characterized in that** it further includes, in parallel, at least one oxygen injection channel (26) extended between the proximal end (22) of the catheter where said channel can be connected to a pressurized oxygen source and an oxygen outlet (25) opening at the periphery of the catheter (2) and set back from the distal end (3) of the catheter, the injection channel (26) and the lumen (24) being independent of each other, said catheter (2) with oxygen injection channel (26) being in particular intended to be used in a breathing assistance device (4) having no oxygen injection means in its main channel (5).

6. The catheter (2) according to claim 5, **characterized in that** the oxygen injection channel (26) is configured so that its oxygen outlet (25) is in the main channel (5), towards the distal end (7) of the breathing assistance device (4).

7. The catheter (2) according to claim 6, **characterized in that** the oxygen outlet of the oxygen injection channel (26) includes at least one orifice (25) creating a jet that is inclined with respect to the external wall of the catheter (2) and that is hence directed so as to hit the wall of the main channel (5) of the breathing assistance device (4).

8. The catheter (2) according to claim 7, **characterized in that** it includes a set of inclined jet orifices (25) distributed over the perimeter of the catheter (2).

9. A breathing assistance device (4) and catheter (2) set (1), **characterized in that** the catheter is a combined measurement catheter (2) according to any one of the preceding claims and **in that** it is removable from the breathing assistance device.

10. The set (1) according to claim 9, **characterized in that** the breathing assistance device (4) is chosen among a facial mask, a tracheal intubation tube (4) or a pharyngeal or laryngeal mask tube.

11. The set (1) according to claim 9 or claim 10, **characterized in that** the catheter (2) is single use and is intended to be connected to a gaseous composition measurement apparatus and to a gaseous pressure measurement apparatus and **in that** the gaseous composition measurement apparatus (16) is removable to be reused in several catheters and the pressure measurement apparatus (15) is single use.
